(19) Europäisches Patentamt

European Patent Office

Office européen des brevets

(11) Publication number: **0 510 770 A2**

# EUROPEAN PATENT APPLICATION

(21) Application number: **92201128.3**

(22) Date of filing: **21.04.92**

(51) Int. Cl.5: **C07C 1/04**, B01J 35/02

(30) Priority: **23.04.91 GB 9108657**

(43) Date of publication of application:
**28.10.92 Bulletin 92/44**

(84) Designated Contracting States:
**BE DE FR GB IT NL**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Reinalda, Donald**
**Badhuisweg 3**
**NL-1031 CM Amsterdam(NL)**
Inventor: **Blankenstein, Paul**
**Badhuisweg 3**
**NL-1031 CM Amsterdam(NL)**

(54) **Process for the preparation of hydrocarbons.**

(57) A process for the preparation of hydrocarbons comprises contacting a mixture of carbon monoxide and hydrogen at elevated temperature and pressure with a catalyst in a reaction zone, which catalyst comprises a catalytically active component and a refractory oxide carrier and is in the form of particles retained in a fixed bed within the reaction zone, which particles are helical lobed particles. The catalyst particles are preferably prepared by extrusion through a suitable dieplate.

FIG. 2

The present invention relates to a process for the preparation of hydrocarbons, in particular to a process for the preparation of hydrocarbons from a mixture of hydrogen and carbon monoxide.

The preparation of hydrocarbons by contacting a mixture of carbon monoxide and hydrogen with a suitable catalyst at elevated temperature and pressure is known in the art as the Fischer-Tropsch synthesis. Recently, much attention has been paid to the development of processes employing the Fischer-Tropsch synthesis in order to prepare hydrocarbons having a high molecular weight and which are present as liquids under the prevailing process conditions.

The Fischer-Tropsch synthesis may be carried out using a variety of reaction regimes, for example a fluidized bed regime or a slurry bed regime. One particular reaction regime which is finding increasing favour in the art is the fixed bed regime, in which the carbon monoxide and hydrogen are contacted with particles of catalyst retained as a fixed bed within the reaction vessel.

A variety of shapes and designs of catalyst particles for use in the fixed bed operation of the Fischer-Tropsch synthesis have been proposed. Thus, European Patent Application No. 90 202989.1 (as yet unpublished) discloses that the catalyst particles may be in the form of cylinders; hollow cylinders, for example cylinders having a central hollow space which has a radius of between 0.1 and 0.4 of the radius of the cylinder; straight or rifled (twisted) trilobes; or one of the other forms disclosed in US patent No. 4,028,221. Trilobe extrudates are said to be preferred.

In a process employing the fixed bed regime, as with other regimes, it is most desirable to employ a highly efficient catalyst. In terms of the Fischer-Tropsch process, a highly efficient catalyst is one which exhibits not only a high level of activity for the conversion of carbon monoxide and hydrogen to hydrocarbons, but also a high degree of selectivity to higher molecular hydrocarbons, in particular $C_5$ hydrocarbons and larger. It is taught in the prior art that the efficiency of a catalyst, in general increases as the size of the catalyst particles decreases.

When using a process employing a fixed bed of catalyst particles, a major consideration in the design of the process is the pressure drop through the catalyst bed. It is most desirable that the pressure drop be as low as possible. However, it is well reported in the art that, for a given shape of catalyst particle, as the size of catalyst particles in a fixed bed is reduced, there is a corresponding increase in pressure drop through the catalyst bed. Thus, there exists a conflict in the design of fixed catalyst beds when trying to achieve a satisfactory level of catalyst efficiency whilst keeping the pressure drop through the bed to a minimum. This is particularly the case in the design of a fixed bed reactor for use in a Fischer-Tropsch synthesis.

European patent No. 0 218 147 (EP-B-O 218 147) discloses a helical lobed, polylobal extrudate particle having the outline shape of three or four strands helically wound about the axis of extrusion along the length of the particle and its use as a catalyst or catalyst support, in particular as a catalyst or support in hydrotreating operations. The helical shape of the catalyst particle is said to reduce the pressure drop across fixed bed reactors through which liquid and/or gas reactants are passed. In this way, smaller catalyst particles can be employed in a given reactor design to meet the pressure drop requirements which can only be met by particles of other designs. The smaller particles are said to improve catalyst efficiency. It is described in EP-B-0 218 147 that the helicial lobed particles, when packed in a bed, result in a greater voidage and, hence, reduced density of catalyst, than equivalent straight lobed particles. In EP-B-0 218 147, it is stated that, when tested for performance in the desulphurisation of petroleum fractions, the helical lobed particles showed no significant difference in catalytic activity to the equivalent straight lobed particles tested under the same reaction conditions, but resulted in a reduced pressure drop across the bed. Literature published by the patentee of EP-B-0 218 147 confirms that, in hydrotreating applications, the volume activity for the helical lobed particles is equal to that of the equivalent straight lobed particles.

Most surprisingly, it has been found that, contrary to the teaching of EP-B-0 218 147, the application of helical lobed catalyst particles in fixed catalyst beds employed in Fischer-Tropsch synthesis results in a substantial increase in the efficiency of the catalyst, for a given volume of fixed bed, compared with the equivalent straight lobed catalyst particles, whilst still giving the advantages arising from a reduction in pressure drop. In particular, the helical lobed catalyst particles exhibit a markedly higher selectivity to higher molecular weight hydrocarbons that the equivalent straight lobed catalyst particles.

Accordingly, the present invention provides a process for the preparation of hydrocarbons, which process comprises contacting a mixture of carbon monoxide and hydrogen at elevated temperature and pressure with a catalyst in a reaction zone, which catalyst comprises a catalytically active component and a refractory oxide carrier and is in the form of particles retained in a fixed bed within the reaction zone, which particles are helical lobed particles.

The process of the invention is particularly advantageous when the hydrocarbons prepared are present in substantial quantity as a liquid under the prevailing reaction conditions.

By employing helical lobed catalyst particles in the process of the present invention, a larger diameter helical lobed catalyst particle can be employed to achieve a given selectivity than is necessary when employing straight lobed particles, resulting in a greater reduction in pressure drop across the catalyst bed than expected from the prior art. Alternatively, for a given design of fixed bed with a predetermined pressure drop, by employing the helical lobed particles in the Fischer-Tropsch process a substantially higher selectivity can be achieved than with the appropriate straight lobed particles necessary to meet the pressure drop requirements.

The term "helical lobed particle" as used herein is a reference to an elongate particle having a cross-section comprising three or four lobes formed by the intersection of respectively three or four elipses, which lobes extend along and are helically wound about the longitudinal axis of the particle. Preferably, the particle has a cross-section comprising lobes formed by the intersection of circles, which particle has a surface in the form of cylinders helically wound about the longitudinal axis of the particles. The particle preferably has three lobes; hereafter referred to as "trilobe".

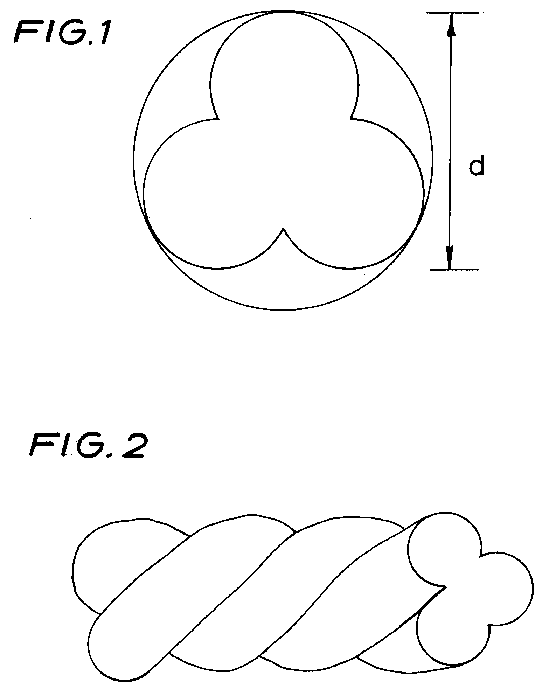

A preferred particle is illustrated in Figures 1 and 2, in which:

Figure 1 is a cross-sectional view of a preferred catalyst particle for use in the process of the present invention; and

Figure 2 is a perspective view of the particle of Figure 1 from one side.

The catalyst particles may have any suitable diameter to meet the design requirements of the process. In particular, the particles may have a nominal diameter in the range of from 0.5 to 3 mm, more preferably from 1 to 2 mm. In this respect, the nominal diameter of a helical lobed particle is to be taken as the length of a line, in a cross-section of the particle, drawn through the centre of the particle and extending from the line tangential to two adjacent lobes to the opposing extremity of the particle, if consisting of three lobes, or to the line tangential to the other two lobes, if the particle consists of four lobes. The nominal diameter of a trilobe is represented as the distance "d" in Figure 1. The helically formed lobes may have a pitch in the range of from 1 to 10 turns of one lobe per 2.5 cm of extrudate length, preferably from 2 to 5 turns per 2.5 cm. The catalyst particles preferably have a length in the range of from 0.5 to 5 cm, preferably 1 to 3 cm.

The catalyst used in the process of the present invention may comprise, as the catalytically active component, any of the elements having activity as catalysts in the Fischer-Tropsch synthesis. In particular, the catalyst may comprise, as the catalytically active component, one or more metals from Group VIIB or Group VIII of the Periodic Table of Elements, preferably Group VIII. More particularly, the catalyst preferably comprises one or more metals from the iron group, namely iron, cobalt and nickel. The catalyst most preferably comprises cobalt.

The catalytically active components may be present in the catalyst together with one or more promoters or co-catalysts. The promoters may be present either as metals or metal oxides. Suitable metal oxide promoters include oxides of metals from Groups IIA, IIIB, IVB, VB, or VIB of the Periodic Table from the lanthanides and/or actinides. Preferably, the catalyst comprises a source of an element in Group IVB of the Periodic Table, in particular titanium or zirconium. Catalysts containing zirconium are particularly preferred. As an alternative or in addition to the metal oxide promoter, the catalyst may comprise a metal promoter selected from the Groups VIIB and/or VIII of the Periodic Table. Preferred metal promoters include platinum and palladium. A most preferred catalyst comprises cobalt as a catalytically active component together with zirconium as a promoter.

The catalyst used in the process of the present invention also comprises a refractory oxide carrier, on which the catalytically active components and promoters are supported. The carrier may be any suitable refractory oxide, for example alumina, silica, titania, zirconia or mixtures thereof. Silica and/or alumina are preferred materials for use as a carrier. Silica is a most preferred carrier material. If a material other than silica is used as the carrier material, the catalyst preferably comprises silica in addition thereto. The aforementioned refractory oxides are available commercially and are well known as carrier materials in the art.

The catalyst particles used in the process of the present invention may be prepared by a process comprising mulling a mixture of the finely divided refractory oxide or refractory oxide precursor and a suitable solvent; extruding the resulting mixture through an orifice in a die; and drying the resulting extrudates.

The solvent for inclusion in the mixture may be any of the suitable solvents known in the art. Examples of suitable solvents include water; alcohols, such as methanol, ethanol and propanol; ketones, such as acetone; aldehydes, such as proponal; and aromatic solvents, such as toluene.

A most convenient and preferred solvent is water.

The mixture being mulled may comprise, in addition to the refractory oxide or refractory oxide precursor and solvent, one or more sources for one or more of the catalytically active components

EP 0 510 770 A2

hereinbefore discussed. Alternatively, or in addition thereto, the mixture may comprise one or more sources for one or more of the aforementioned promoters.

The source for the catalytically active component and/or the promoter may be either soluble or insoluble in the solvent. Typical sources include salts derived from organic acids, for example acetates, benzoates, ethanoates and propionates; halides, for example chlorides, bromides, iodides and fluorides; and other salts, for example nitrates, oxides, hydroxides, carbonates and chlorates. Sources insoluble in the solvent are preferred. Hydroxides have been found to be particularly suitable sources for the catalytically active elements and the promoters.

To obtain strong extrudates, it is preferred to include in the mixture a suitable peptizing agent for the refractory oxide or refractory oxide precursor. Suitable peptizing agents for silica, titania and zirconia are basic compounds. The basic compound is preferably ammonia, an ammonia-releasing compound, an ammonium compound or an organic amine. Such basic compounds are removed upon calcination and are not retained in the extrudates to impair the catalytic performance of the final product. The basic compound is most preferably an organic amine or an ammonium compound. A most suitable organic amine is ethanol amine. It has been found most convenient to include in the mixture a compound of a required element from the aforementioned groups of the Periodic Table, which element is active as a catalytically active component or a promoter, to act as both a source for that element and as the basic compound. In particular, it is preferred to include a source of an element acting as a promoter as the basic compound, most preferably a basic compound of an element in Group IVB of the Periodic Table. A preferred group of basic compounds are the ammonium carbonates. Ammonium zirconium carbonate is a particularly preferred basic compound for inclusion in the mixture.

A basic compound of a catalytically active component and/or a promoter may be used in addition to both one or more of the aforementioned sources of that element and a basic compound. However, it has been found possible to dispense with both a separate source for the desired element and an additional basic compound by using a basic compound of the desired element.

The amount of basic compound included in the mixture should be sufficient to peptize the silica, titania or zirconia or their precursors present in the mixture. The amount of basic compound present in the mixture can be readily determined by measuring the pH of the mixture. During mulling the mixture should preferably have a pH in the range of from 8.0 to 11.5, preferably from 9.0 to 11.0.

Alternatively, if alumina is used as the refractory oxide, suitable peptizing agents for the alumina are acidic compounds. Suitable acidic compounds include both organic and inorganic acids and salts thereof. Suitable inorganic acids include aqueous solutions of hydrogen fluoride, hydrogen chloride and hydrogen bromide, nitric acid, nitrous acid and perchloric acid. Preferably, an organic acid is used as the peptizing agent, for example an alkanoic acid having from 1 to 12 carbon atoms, more preferably from 1 to 6 carbon atoms, or a dicarboxylic acid having from 1 to 6 carbon atoms. Particularly preferred acids are the alkanoic acids, especially formic acid, acetic acid, propionic acid and butanoic acid. Acetic acid is particularly preferred.

The amount of acidic compound included in the mixture should be sufficient to peptize the alumina or alumina precursor, and can be readily determined by measuring the pH of the mixture. During mulling the mixtures should preferably have a pH in the range of from 1 to 6, preferably from 4 to 6.

It has been found that the extrudates produced by the extrusion of mixtures having a high or low pH may be less satisfactory than those produced by the extrusion of mixtures having a pH in the range of from 6.0 to 8.0. Accordingly, to avoid extruding a mixture having a high or low pH, it may be preferred to alter the pH of the mixture, after allowing sufficient time for the refractory oxide or refractory oxide precursor to be peptized by the basic compound, but before extrusion, to a pH value in the range of from 6.0 to 8.0. The reduction in pH from a high pH may be effected by the addition of an acid. Suitable acids include both organic and inorganic acids. Suitable inorganic acids include aqueous solutions of hydrogen fluoride, hydrogen chloride and hydrogen bromide, nitric acid, nitrous acid and perchloric acid. Preferably, an organic acid is used, for example an alkanoic acid having from 1 to 12 carbon atoms, preferably from 1 to 6 carbon atoms, or a dicarboxylic acid having from 1 to 6 carbon atoms. Particularly preferred acids are the alkanoic acids, especially formic acid, acetic acid, propionic acid and butanoic acid. Acetic acid is most preferred. An increase in pH from a low pH value may be effected by the addition to the mixture of a suitable basic compound. Suitable basic compounds include both inorganic and organic bases. Inorganic bases which may be used are, for example, alkali or alkaline earth metal hydroxides, such as sodium hydroxide, potassium hydroxide and magnesium hydroxide. Suitable organic bases include amines, for example ethylamine and propylamine, and alkali metal alkoxides, for example sodium methoxide and sodium ethoxide.

To improve the flux properties of the mixture during extrusion a surface active agent or polyelectrolyte

4

may be added to the mixture. The addition of the surface active agent results in a smoother extrusion, a smoother extrudate texture and facilitates cutting of the extruded product. Further, the formation of pores in the calcined catalytic material may be improved which may enhance the catalytic properties of the products. Suitable surface active agents include cationic surface active agents, for example fatty amines, quaternary ammonium compounds, aliphatic monocarboxylic acids, ethoxylated alkyl amines, polyvinyl pyridine, sulphoxonium, sulphonium, phosphonium and iodonium compounds; anionic surface active agents, for example alkylated aromatic compounds, acyclic monocarboxylic acids, fatty acids, sulphonated aromatic compounds, alcohol sulphates, ether alcohol sulphates, sulphated fats and oils and phosphonic acid salts; and nonionic surface active agents, for example polyethylene alkylphenols, polyoxyethylene alcohols, polyoxyethylene alkylamines, polyoxyethylene alkylamides, polyols and acetylenic glycols. The amount of surface active agent is typically from 0.5 to 8% by weight, preferably from 1 to 5% by weight, based on the weight of refractory oxide in the mixture. A preferred surface active agent is sold under the trademark Nalco. The surface active agent may be added at any stage during the mulling of the mixture prior to extrusion, typically just prior to extrusion.

In the process of the present invention, a mixture of the aforementioned components is mulled. In principle, it is possible to combine the components of the mixture in any order. However, it has been found advantageous to prepare and mull the mixture in the following manner. At the very least, the mixture comprises a refractory oxide or refractory oxide precursor and a solvent, which are first mixed together. If the mixture is to include an acidic or a basic compound, it has been found advantageous to add the compound to the mixture after the refractory oxide or refractory oxide precursor and solvent have been combined. In this way, the considerable take up of the basic compound into the pores of the refractory oxide or refractory oxide precursor is avoided, resulting in improved extrudates. Acidic or basic compound confined within the pores of the refractory oxide or refractory oxide precursor is prevented from fully peptizing the material, requiring the addition of further amounts of the compound for satisfactory peptization, or yielding extrudates having a lower crush strength. The resulting mixture of refractory oxide or refractory oxide precursor, solvent and basic compound may be mixed and mulled. Thereafter, once the refractory oxide or refractory oxide precursor has been satisfactorily peptized, the pH of the mixture may be increased or reduced as hereinbefore described. If desired, a source, of one or more catalytically active components and/or promoters may be added and the resulting mixture subjected to further mulling. A surface active agent, if desired, may be added at any time during the mulling, preferably just prior to a final period of mulling.

Typically, the mixture is mulled for a period of from 10 to 120 minutes, preferably from 15 to 90 minutes. During the mulling process, energy is input into the mixture by the mulling apparatus. The rate of energy input into the mixture is typically from 0.05 to 50 Wh/min/kg, preferably from 0.5 to 10 Wh/min/kg. The mulling process may be carried out over a broad range of temperature, preferably from 15 to 50 °C. As a result of the energy input into the mixture during the mulling process, there will be a rise in temperature of the mixture during the mulling. The mulling process is conveniently carried out at ambient pressure. Any suitable, commercially available mulling machine may be employed.

Once the mulling process has been completed, the resulting mixture is then extruded. Extrusion may be effected using any conventional, commercially available extruder. In particular, a screw-type extruding machine may be used to force the mixture through orifices in a suitable dieplate to yield extrudates having the helical lobed form. The strands formed upon extrusion may be cut to the desired length.

After extrusion, the extrudates are dried. Drying may be effected at an elevated temperature, preferably up to 800 °C, more preferably up to 300 °C. The period for drying is typically up to 5 hours, preferably from 30 minutes to 3 hours.

Preferably, the extrudates are calcined after drying. Calcination is effected at an elevated temperature, preferably up to 1000 °C, more preferably from 200 °C to 1000 °C most preferably from 300 °C to 800 °C. Calcination of the extrudates is typically effected for a period of up to 5 hours, preferably from 30 minutes to 4 hours.

Once prepared, the extrudates may be subjected to a deposition stage in which sources of one or more of the aforementioned catalytically active components and/or promoters are deposited on the extrudates. In cases in which the original mixture comprised a source for a given element, the deposition of a further source for that element may be effected to increase the loading of the element on the extrudates.

Deposition of a source of a catalytically active component or a promoter on the extrudates may be effected by any of the techniques known in the art, for example precipitation. If deposition of sources of a plurality of components on the extrudate is to be effected, the deposition may be carried out in either one or a plurality of stages. The order of deposition of the plurality of sources of the elements is largely a matter of choice and convenience. However, the preferred order is first to deposit one or more promoter sources on the extrudates and secondly to deposit sources of one or more catalytically active components.

A preferred technique for the deposition is impregnation. Impregnation may be effected by contacting the extrudates with a compound of the desired element in the presence of a liquid, preferably in the form of a solution of a compound of the element. Suitable liquids for use in the impregnation include both organic and inorganic liquids, water being a most convenient and preferred liquid. Suitable compounds of the desired element include both organic and inorganic compounds, with a preference for compounds which are soluble in the selected solvent. Preferably, the compounds are inorganic compounds. Nitrates are most preferred compounds.

The extrudates are most conveniently contacted with the compound of the desired element by immersion in the liquid. Preferably, the extrudates are immersed in a sufficient volume of liquid so as to fill the volume of pores in the extrudates.

If the impregnation is conducted in a single stage, the extrudates are contacted simultaneously with a compound of each of the desired elements in the presence of the liquid. Preferably, the extrudates are immersed in an aqueous solution of nitrates of the desired elements. If the impregnation is conducted in a plurality of stages, the extrudates are contacted in a first stage with a compound of one of the desired elements in the presence of a liquid and in a subsequent stage with a compound of a further desired element in the presence of a liquid. The liquid may be the same or different in the stages; most conveniently the same.

After the impregnation, if in a single stage, or after each impregnation in a multi-stage impregnation, the extrudates are dried. The conditions under which the extrudates are dried are those as hereinbefore described. Preferably, after the or each drying process, the extrudates are calcined, the calcination conditions being those as hereinbefore described.

A catalytically active component may be present in the catalyst in an amount of from 1 to 100 parts by weight, preferably 10 to 50 parts by weight, per 100 parts by weight of refractory oxide. The promoter, if present, may be present in an amount of from 1 to 60 parts by weight, preferably from 2 to 40 parts by weight, per 100 parts by weight of refractory oxide.

Prior to being used in the process of the present invention, the catalyst is first activated. Activation may be effected by reducing the catalyst through contact with a hydrogen-containing gas at an elevated temperature. Reduction is preferably effected by contact with the hydrogen-containing gas at a temperature of up to 400 °C, more preferably up to 300 °C, at a pressure of up to 20 bar, more preferably up to 10 bar, and a gas hourly space velocity of from 100 to 10000 Nl/l/h, preferably from 200 to 6000 Nl/l/h. Processes for the activation of Fischer-Tropsch catalysts are known and described in the art.

Thereafter, the resulting activated catalyst is contacted with a mixture of carbon monoxide and hydrogen at an elevated temperature and pressure. Typically, the reaction is effected at a temperature in the range of from 125 to 350 °C, preferably from 175 to 250 °C. The reaction pressure is typically in the range of from 5 to 100 bar, preferably from 12 to 50 bar. The hydrogen/carbon monoxide molar ratio in the feed gas is typically greater than 1.5, preferably between 1.75 and 2.25. Unconverted hydrogen and carbon monoxide may be recycled to again contact the catalyst. In such an arrangement, the molar ratio of hydrogen to carbon monoxide in the gas actually contacting the catalyst may be considerably lower than that of the feed gas for example in the range of from 0.9 to 1.3, preferably about 1.1.

Dieplates for use in the extrusion apparatus for use in preparing the helical lobed catalyst particles featuring in the process of the present invention may be prepared by any of the techniques known in the art. Thus, dieplates for use in preparing helical lobed particles having relatively large nominal diameters, that is dieplates having helical orifices with a diameter greater than about 3 mm, may be prepared by conventional machining techniques. For dieplates for use in preparing helical lobed particles having nominal diameters smaller than about 3 mm, it is necessary to employ alternative techniques.

One suitable method, disclosed in EP-B-0 218 147, comprises moulding a thermoplastic material around one or more helical lobed pins in a suitable mould. Suitable thermoplastic materials for use in forming the dieplate include high density polyethylene, polypropylene, thermoplastic acetal resins, some nylons, polycarbonates and the like. Preferred materials include the thermoplastic polyoxymethylene type acetal resin sold under the trademark Delrin and the acetal resin sold under the trademark Celanese Celcon. Once the thermoplastic material has hardened, the pins are removed from the orifices in the dieplate and the mould by being unscrewed. The completed dieplate is then removed from the mould. The pins for use in this process may be prepared by winding three or four strands of wire together to form a wire rope of the appropriate pitch. The rope is then cut to form pins of the desired length. Alternatively, the pins may be prepared by conventional machining and drawing techniques or by electrochemical means.

European Patent Application Publication No. 0 291 682 (EP-A-0 291 682) discloses an improved method for forming dieplates having one or more helically shaped extrusion holes extending through the dieplate, in which a thermoplastic material is placed in a mould in which are retained a plurality of helical lobed pins, each pin being mounted to the base of the mould by a mounting means which retains the pin in the mould whilst allowing it to rotate about its longitudinal axis. To remove a formed and hardened dieplate from the mould, the dieplate is withdrawn from the mould whilst, at the same time, allowing or causing each of the pins to rotate and thus unscrew from the orifice in the hardened dieplate.

However, it is now been found possible to prepare the dieplates by punching helical lobed orifices directly into a hardened blank of suitable material. Preferably, the dieplates are formed using a method employing a novel punching apparatus to punch the required orifices directly into a blank. The novel punch apparatus for use in this method comprises a punch assembly movable along a line and comprising a first member fixed about the line; a second member rotatable about the line; and means retaining the second member in a fixed position in the line with respect to the first member, the second member having an elongate, helical lobed punching portion having a longitudinal axis coincident with the line, whereby, upon movement of the punch assembly along the line causing the helical lobed punching portion to pass into a yieldable material placed in the line, passage of the helical lobed punching portion of the second member through the material effects rotation of the second member about the line.

The term "helical lobed" as used herein is a reference to the form of the surface of the helical lobed particle, which term has been defined hereinbefore. References to a "helical lobed orifice" are references to orifices having an inside surface which is helical lobed in form.

The term "yieldable material" is a reference to any material which yields under the movement of the punching portion allowing the punch to pass through the material and may be any of the materials discussed hereinbefore as being suitable materials for use in preparing extrusion dieplates.

The second member may consist of a single component, appropriately formed so as to comprise a helically lobed portion to act as a punch. Preferably, however, the second member comprises a punch mounting means, in which case the helically lobed punching portion of that member comprises a suitable punch located in the mounting means. In this way, a variety of forms and sizes of punch can be employed in a single punch apparatus. Conveniently, the punch mounting means comprises a bore in the second member into which the appropriate punch can be inserted and secured so as to project from the bore.

The means for retaining the second member preferably comprises a housing extending from the first member to define a chamber with the first member, the housing having an inner bearing surface opposing a bearing surface on the first member, the aforesaid bearing surfaces lying in planes perpendicular to the line; the second member having a bearing portion laying within the housing between the aforesaid bearing surfaces and having a bearing surface opposing each of the aforesaid bearing surfaces; the second member extending through an apperture in the housing with the helically lobed punching portion lying at least partially outside the housing.

The housing may be unitary with the first member or formed as a separate component and mounted on the first member.

It will be readily appreciated that the second member must be able to rotate freely about the line of movement with little friction. This may be achieved by the selection of appropriate materials having low coefficient of friction and by preparing the opposing surface. In this way, the opposing bearing surfaces may contact one another directly, with an appropriate distance being left to ensure the smooth running of one surface over another. Preferably, a bearing is provided between the inner bearing surface of the housing and the opposing surface of the bearing portion of the second member or between the bearing surface on the first member and the opposing surface of the bearing portion of the second member.

More preferably, a bearing is provided between each of the bearing surfaces of the bearing portion of the second member and the opposing surface. The bearing is very suitably a ball or roller bearing.

The second member may be left free to rotate and adopt any orientation about the line of movement. However, it is preferred to provide a biassing means biassing the second member to a predetermined orientation about the line. The biassing means is preferably a resilient biassing means, very conveniently a spring, preferably a tension spring. A preferred arrangement is one in which the biassing means is a resiliant biassing means extending between a first biassing means mounting rigidly connected to the first member and a second biassing means mounting connected to the second member; the predetermined orientation of the second member about the line being determined by a stop rigidly connected to the first member and interacting with the second biassing means mounting. The rigid connection of the first biassing means mounting and the stop to the first member is conveniently by means of the housing.

The helical lobed punching portion of the second member is preferably in the form of a helical trilobe.

Accordingly, there is further provided a method of forming a helical lobed orifice in a yieldable material comprising moving a helical lobed punch in a forward direction into the body of the material; allowing the punch to rotate under the action of the helical lobed punch moving through the material; and moving the helical lobed punch in the reverse direction whilst still allowing the punch to rotate under the action of the helical lobed punch moving through the material.

Preferably, the rotation of the punch whilst movement is in the forward direction is against the bias of a biassing means.

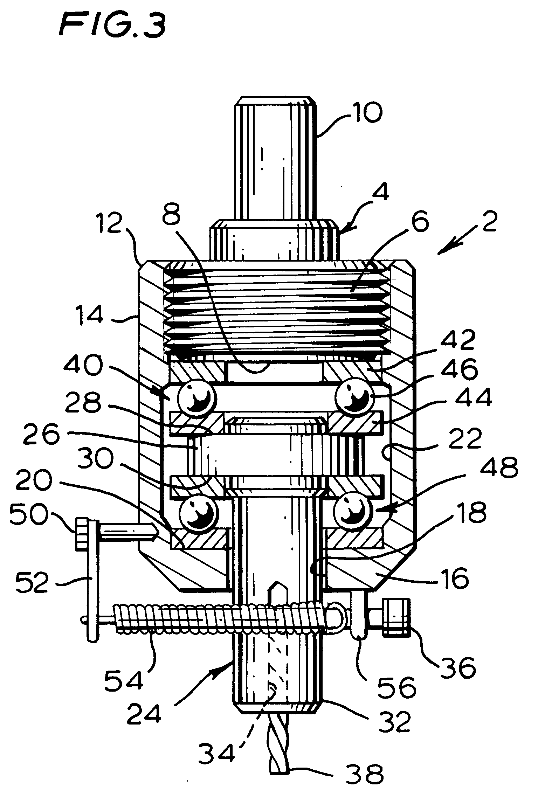

The novel punch apparatus and the method will be further described by way of illustration only, having reference to Figure 3 which is a partial cross-section view of a punch assembly according to the present invention.

Referring to Figure 3, a punch assembly 2 comprises a first member 4 having a cylindrical bearing portion 6, the curved surface of which has been machined to form a thread. The cylindrical bearing portion 6 has a bearing surface 8 disposed at one end. The first member 4 has a shank 10 extending from its opposite and which may be inserted into the chuck or mounting of a conventional punch or press (not shown).

A generally tubular housing 12 bears an internal thread at one end portion 14 which has been threaded onto the cylindrical bearing portion 6 of the first member 4. At its opposite end, the housing 12 has an inwardly extending flange 16 forming an aperture 18 in the housing.

The flange 16 has an inner surface 20 opposing the bearing surface 8 of the cylindrical bearing portion 6 of the first member 4 which, together with the inner surface 22 of the housing defines a chamber. A second member 24 has a bearing portion 26 retained within the chamber, the bearing portion 26 having a first bearing surface 28, opposing the bearing surface 8 of the cylindrical bearing portion 6 of the first member 4, and a second bearing surface 30, opposing the inner surface 20 of the flange 16. The second member 24 further comprises a cylindrical punch-holding shank 32 extending from the centre of the second bearing surface 30 of the bearing portion 26, which shank 32 extends through and projects outwardly from the aperture 18 in the flange 16. A blind bore 34 having a longitudinal axis coincident with the longitudinal axis of the punch-holding shank 32 extends inwardly from the free end of the shank 32. A second bore (not shown) extends radially inwards from the curved surface of the punch holding shank 32 and communicates with the blind bore 34. The second bore is formed with a thread and retains a punch locking screw 36. A helical trilobe punch 38 is retained in the blind bore 34 by the end of the punch locking screw 36. A portion of the helical trilobe punch 38 projects from the blind bore 34.

The second member 24 is retained in a fixed position with respect to the first member 4 by two bearings within the chamber. A first bearing, generally indicated as 40, extends between the first member 4 and the second member 24 and comprises two bearing rings 42, 44, one bearing ring 42 being adjacent the bearing surface 8 of the bearing portion 6 of the first member 4, and the second bearing ring 44 being adjacent the first bearing surface 28 of the bearing portion 26 of the second member 24. Balls 46 are disposed between the two bearing rings 42, 44 and run in grooves formed in the opposing surfaces of the two rings 42, 44. A second bearing, generally indicated as 48, identical to the first bearing 40, extends between the second bearing surface 30 of the bearing portion 26 of the second member 24 and the inner surface 20 of the flange 16.

A spring mounting screw 50 is threaded into a bore in the side of the housing 12. A spring mounting bar 52 rigidly connected to the exposed shank of the spring mounting screw 50 extends parallel to the longitudinal axis of the punch-holding shank 32 in the direction of the punch 38. A coil spring 54 is secured at one end to the free end of the spring mounting bar 52 and at the other end to the exposed shank of the punch locking screw 36. A stop 56 depends from the outer surface of the flange 16 so as to interact with the exposed shank of the punch locking screw 36. The stop 56 and spring 54 are so disposed as to define a rest position in which the punch locking screw 36 is held in contact with the step by the spring 54 which is partially extended and in tension.

In operation, the punch assembly shown in Figure 3 is mounted in a suitable punch or press apparatus. The assembly is moved into a position such that the projecting portion of the punch 38 contacts and passes into the material to be drilled (not shown), typically a blank extruder dieplate. As the punch 38 enters the material, the helical lobed form of the punch interacting with and shaping the material causes the punch 38 and, hence, the second member 24 to rotate. Rotation of the punch 38 and the second member 24 is against the bias of the spring 54, the tension in which is increased as the second member 24 rotates and the punch locking screw 36 is moved out of contact with the stop 56. Once the material has been penetrated by the punch 38 to the required depth, the direction of movement is reversed to remove the punch 38 from the material. Upon reversal, the direction of rotation of the second member 24 is reversed and the punch assembly returns, upon removal, to the rest position. The process is preferably repeated one or more times.

It is preferable that the material to be punched is preformed or machined so as to have a bore or orifice already present at the position in which the helical lobed orifice is required, which bore or orifice is of smaller diameter than the nominal diameter of the punch. In this respect, the term "nominal diameter" of a helical lobed punch is as hereinbefore defined with reference to the helical lobed catalyst particles.

Dieplates for use in an extrusion apparatus are typically cylindrical in form and have a diameter of 1.5 to 2 cm and a length of 5 to 10 mm. The number of orifices in a single dieplate may vary and is typically from about 10 to about 20.

The process of the present invention is further described in the following illustrative example. In the example, values for the loss on ignition are quoted on the basis of water lost upon heating the sample to a temperature in the range of from 550 to 600 °C. Calcination was conducted at the temperature and for the duration quoted in an atmosphere of air.

EXAMPLE

(i) Extrudate Preparation

A mixture comprising silica (precipitated silica, average particle size 50 $\mu$m, surface area 450 $m^2/g$; 2257 g), ammonium zirconium carbonate (Bacote 20, 20 %wt equivalent of $ZrO_2$, 1661 g) and water (2400 g) was mulled for a period of 20 minutes. Acetic acid (5% aqueous solution, 116 g) and water (702 g) were added and the resulting mixture mulled for a further 30 minutes. Polyelectrolyte (Nalco) (93 g as a 4% aqueous solution) was added and the resulting mixture mulled for a further 5 minutes to yield a final mixture having a pH of 8.4 and a loss as ignition of 69.8%.

A 2.0 mm helical trilobe Delrin matrix dieplate was prepared using the apparatus shown in Figure 3 and the method as hereinbefore described. The dieplate was inserted in a 2.25 inch Bonnot extruder and used to extrude the mixture prepared as described above. The resulting helical trilobe extrudates were dried at a temperature of 120 °C and finally calcined at a temperature of 800 °C for 2 hours. The resulting extrudates had a nominal diameter of 1.5 mm, high crush strength and a pore volume ($H_2O$) of 0.81 ml/g.

(ii) Catalyst Preparation

The extrudates prepared in (i) above (21.09 g) were washed by stirring in an aqueous solution of ammonium acetate (1M; 106 ml) for a period of 30 minutes. The extrudates were removed by filtration and the washing repeated a second time in fresh ammonium acetate solution (1M; 52.5 ml). The extrudates were again removed by filtration and the second washing treatment repeated again. After removal from the final washing solution, the extrudates were calcined by heating from 100 °C to a temperature of 500 °C and being held at 500 °C for 1 hour.

An aqueous solution was prepared by dissolving cobalt nitrate ($Co(NO_3)_2.6H_2O$, 88.88 g) in water (sufficient for a total solution weight of 100.13 g) and heating to a temperature of 80 °C. The extrudates (17.59 g) were impregnated by mixing with the cobalt nitrate solution (14.3 ml; 23.74 g) at 80 °C. The extrudates remained in the solution for 8 hours at 80 °C. The thus impregnated extrudates were then dried and finally calcined by heating to a temperature of 500 °C and being held at 500 °C for 2 hours.

(iii) Catalyst Testing

The catalyst prepared in (ii) above was loaded into a reaction vessel. The catalyst was first activated by reduction by being contacted with a mixture of hydrogen and nitrogen at a temperature of 250 °C, a pressure of 5 bar and a gas hourly space velocity of from 500 to 600 Nl/l/h. The activated catalyst was then contacted with a mixture of carbon monoxide and hydrogen having a hydrogen/carbon monoxide ratio of 1.1 at a gas inlet pressure of from 37 to 39 bar and a gas hourly space velocity of from 1110 to 1130 Nl/l/h for a period of 100 hours. The resulting catalyst was then reduced by contact with a mixture of hydrogen and nitrogen at a temperature of 250 °C, a pressure of 10 bar and a gas hourly space velocity of 700 Nl/l/h for a period of 24 hours. The thus reduced catalyst was then oxidized by contact with a mixture of oxygen (0.5 %v) and nitrogen at a temperature of 250 °C, a pressure of 10 bar and a gas hourly space velocity of 700 Nl/l/h for a period of 24 hours. Finally, the catalyst was again reduced in a mixture of hydrogen and nitrogen under the aforementioned conditions. The catalyst was then contacted with a mixture of carbon monoxide and hydrogen at the pressure, space velocity and hydrogen/carbon monoxide ratio mentioned above. The reactor temperature necessary to achieve a space time yield of 100 g product per litre of catalyst per hour (100 STY Temperature) and the selectivity of the catalyst to hydrocarbons having 5 carbon atoms or more ($C_5^+$ selectivity) is shown in Table I.

Comparative Examples

Following the general procedure outlined in the above Example, two further batches of catalyst, hereafter catalysts A and B, were prepared having an equivalent loading of cobalt and zirconium on the silica carrier using a variety of straight trilobe Delrin matrix dieplates inserted into the Bonnot extruder. The nominal diameters of the resulting catalysts A and B are as follows:

| Catalyst | Nominal Diameter (mm) |
|---|---|
| A | 1.87 |
| B | 1.71 |

Catalysts A and B were each packed into an identical reactor as in section (iii) of the Example and activated and tested under the conditions set out in section (iii) of the above Example. The 100 STY Temperatures and the $C_5^+$ selectivity of catalysts A and B are set out in Tables II and III respectively.

| Run time (hours) | 100 STY Temperature (°C) | $C_5^+$ selectivity (%wt) |
|---|---|---|
| 35 | 213.0 | 91.25 |
| 44 | 212.5 | 91.75 |
| 48 | 213.5 | 91.2 |
| 124 | 214.5 | 91.4 |
| 177 | 215.0 | 91.75 |

Table I 1.5 mm Helical Trilobes

| Run time (hours) | 100 STY Temperature (°C) | $C_5^+$ selectivity (%wt) |
|---|---|---|
| 125 | 221.0 | 88.25 |
| 140 | 220.0 | 88.2 |
| 160 | 220.5 | 88.0 |
| 195 | 220.0 | 88.4 |

Table II Catalyst A; 1.87 mm Straight Trilobes

10

| Run time (hours) | 100 STY Temperature (°C) | $C_5^+$ selectivity (%wt) |
|---|---|---|
| 38 | 213.0 | 90.25 |
| 45 | 213.0 | 90.25 |
| 55 | 213.0 | 90.4 |
| 112 | 215.5 | 90.2 |
| 135 | 215.5 | 90.6 |
| 145 | 216.5 | 90.2 |
| 162 | 216.0 | 90.2 |
| 170 | 215.5 | 90.6 |

<u>Table III</u>  Catalyst B; 1.71 mm Straight Trilobes

It can be clearly seen from the data set out in Tables I to III that the variation in catalytic activity of the straight trilobes with varying particle diameter follows the pattern taught in the prior art discussed hereinbefore, that is, the activity of the catalyst increases with decreasing diameter of the catalyst particle, as evidenced by a reduction in the 100 STY Temperature. This pattern is repeated in the variation in the selectivity of the straight trilobes with varying diameter, that is selectivity increases as the particle diameter decreases.

However, it can also be seen that, for a given particle diameter, the helical trilobes exhibit a markedly superior selectivity to higher hydrocarbons ($C_5^+$ selectivity) than the straight trilobes.

**Claims**

1. A process for the preparation of hydrocarbons, which process comprises contacting a mixture of carbon monoxide and hydrogen at elevated temperature and pressure with a catalyst in a reaction zone, which catalyst comprises a catalytically active component and a refractory oxide carrier and is in the form of particles retained in a fixed bed within the reaction zone, which particles are helical lobed particles.

2. A process according to claim 1, wherein the hydrocarbons are present in substantial quantities as a liquid.

3. A process according to either of claims 1 or 2, wherein the catalyst particles are trilobes.

4. A process according to any preceding claim, wherein the catalyst particles have a nominal diameter of from 0.5 to 3 mm, preferably from 1 to 2 mm.

5. A process according to any preceding claim, wherein the catalyst comprises, as a catalytically active component, a metal from Group VIII of the Periodic Table, preferably iron, cobalt or nickel, more preferably cobalt.

6. A process according to any preceding claim, wherein the catalyst comprises a promoter selected from oxides of metals in Groups IIa, IIIB, IVB, VB and VIB of the Periodic Table, the lanthanides and actinides, and metals in Groups VIIB and VIII of the Periodic Table, preferably an oxide of a metal in Group IVB of the Periodic Table, more preferably zirconium oxide.

7. A process according to any preceding claim, wherein the refractory oxide is selected from alumina, silica, titania, zirconia and mixtures thereof, preferably silica.

8. A process according to any preceding claim, wherein the mixture of carbon monoxide and hydrogen is contacted with the catalyst at a temperature in the range of from 125 to 350 °C.

9. A process according to any preceding claim, wherein the mixture of carbon monoxide and hydrogen is contacted with the catalyst at a pressure in the range of from 5 to 100 bar.

10. A process according to any preceding claim, wherein the molar ratio of hydrogen to carbon monoxide is in the range of from 0.9 to 1.3.

11. Hydrocarbons prepared by a process according to any preceding claim.

## FIG.1

d

## FIG.2

FIG.3